# NEUE EUROPÄISCHE PATENTSCHRIFT

(11) **EP 2 110 110 B2**
(45) Veröffentlichungstag und Bekanntmachung des Hinweises auf die Entscheidung über den Einspruch: **01.04.2020**
(45) Hinweis auf die Patenterteilung: 27.07.2016
(21) Anmeldenummer: 09005036.0
(22) Anmeldetag: 06.04.2009
(51) Int. Cl.: A61F 13/62, A61F 13/58

(54) **Windelverschlussband, Windel, Halbzeugstreifen und Herstellverfahren für einen Halbzeugstreifen**
Diaper fastener, nappy, semi-finished strips and production method for a semi-finished strip
Bande de fermeture de couche, couche, produit semi-fini et procédé de fabrication pour le produit semi-fini

(30) Priorität: 15.04.2008 DE 102008019035
(43) Veröffentlichungstag der Anmeldung: 21.10.2009
(73) Patentinhaber: Lohmann-koester GmbH & Co. KG, 96146 Altendorf (DE)
(72) Erfinder: Neugebauer, Robert, 91077 Neunkirchen (DE); Degelmann, Walter, Dr., 95326 Kulmbach (DE); Schindler, Udo, 91054 Erlangen (DE)
(74) Vertreter: Reuther, Martin

(56) Entgegenhaltungen:
- EP-A1- 1 000 598
- EP-A1- 1 598 037
- EP-A1- 1 872 760
- EP-A2- 0 853 935
- EP-A2- 0 925 770
- EP-B1- 1 286 635
- WO-A1-92/01401
- WO-A1-98/22069
- WO-A1-2005/060912
- WO-A1-2007/123445
- WO-A2-02/26182
- US-A- 5 605 729
- US-A- 6 063 466
- US-A1- 2008 009 816
- US-A1- 2008 016 656
- US-B1- 6 363 587

## Beschreibung

Die Erfindung betrifft einerseits ein Windelverschlussband mit einer Befestigungsseite und mit einer Rückseite, wobei auf der Befestigungsseite in wenigstens einem Befestigungsgebiet eine Komponente eines zweikomponentigen Verschlusssystems vorgesehen ist, die auf einer Trägerbahn angeordnet ist. Ebenso betrifft die Erfindung eine mit einem derartigen Windelverschlussband versehene Windel. Darüber hinaus betrifft die Erfindung einen Halbzeugstreifen umfassend eine Trägerbahn mit einer Befestigungsseite und mit einer Rückseite, bei welchem auf der Befestigungsseite der Trägerbahn eine Komponente eines zweikomponentigen Verschlusssystems angeordnet ist bzw. bei welchem auf der Befestigungsseite in wenigstens einem Befestigungsgebiet eine Komponente eines zweikomponentigen Verschlusssystems vorgesehen ist, die auf einer Trägerbahn angeordnet ist, und die Trägerbahn auf ihrer Befestigungsseite eine dehäsive Beschichtung aufweist. Auch betrifft die Erfindung ein Verfahren zum Applizieren eines Windelverschlussbandes an einer Windel, wobei das Windelverschlussband von einem Habzeugstreifen senkrecht zu einer Maschinenrichtung abgetrennt und an der Windel appliziert wird, sowie ein Verfahren zum Herstellen eines entsprechenden Halbzeugstreifens mit einer Befestigungsseite und mit einer Rückseite, wobei auf der Befestigungsseite in wenigstens einem Befestigungsgebiet eine Komponente eines zweikomponentigen Verschlusssystems vorgesehen ist, die über eine Klebstoffschicht mit einer Trägerbahn, welche auf der Befestigungsseite mit einer dehäsiven Beschichtung vorgesehen ist, verbunden ist.

Es ist Aufgabe vorliegender Erfindung, ein Windelverschlussband bereitzustellen, welches insbesondere im Bereich eines Befestigungsgebietes eine außergewöhnlich angenehme Haptik gewährleistet und dennoch aus einem möglichst einfach aufgebauten Halbzeug gefertigt werden kann. Als Lösung werden Windelverschlussbänder, Windeln, Halbzeugstreifen, Applikationsverfahren für ein Windelverschlussband und Herstellverfahren für einen Halbzeugstreifen mit den Merkmalen der unabhängigen Ansprüche vorgeschlagen.

So kann sich ein Windelverschlussband mit einer Befestigungsseite und mit einer Rückseite bei welchem auf der Befestigungsseite in wenigstens einem Befestigungsgebiet eine Komponente eines zweikomponentigen Verschlusssystems vorgesehen ist, die auf einer Trägerbahn angeordnet ist, dadurch auszeichnen, dass die Trägerbahn zumindest hinter einem Teilgebiet des Befestigungsgebietes mehrlagig ausgebildet ist, um ein Windelverschlussband bereitzustellen, welches insbesondere im Bereich eines Befestigungsgebietes eine außergewöhnlich angenehme Haptik gewährleistet und dennoch aus einem möglichst einfach aufgebauten Halbzeug gefertigt werden kann.

Dadurch, dass die Trägerbahn zumindest teilweise hinter dem Teilgebiet des Befestigungsbereiches mehrlagig ausgebildet ist, kann das erfindungsgemäße Windelverschlussband einerseits besonders robust an einer Windel appliziert werden. Andererseits erwächst aus einer derartigen Mehrlagigkeit der Trägerbahn die gewünschte Haptik hinsichtlich des Windelverschlussbandes.

Der Begriff "Befestigungsseite" beschreibt vorliegend diejenige Seite einer Baugruppe, insbesondere der Trägerbahn, an welcher sich die Komponente des zweikomponentigen Verschlusssystems befindet. Sollten irgendwelche Umfaltungen oder ähnliches vorliegen, so ändert sich entsprechend die Bezeichnung der gegenüber der Komponente des zweikomponentigen Verschlusssystems umgefateten Bereiche.

Das "Befestigungsgebiet" wird in vorliegendem Zusammenhang durch das Wirkungsgebiet der Komponente des zweikomponentigen Verschlusssystems definiert, wobei das Befestigungsgebiet in der Regel zumindest dort vorliegt, wo diese Komponente vorgesehen ist. Dieses Gebiet der Trägerbahn wird häufig auch als Fasteningbereich bezeichnet. Insoweit ist das Befestigungsgebiet ein Flächengebilde, welches durch die äußere Umrandung der Komponente des zweikomponentigen Verschlusssystems und dessen Wirkfläche definiert wird.

Entsprechend ist die Rückseite eine Baugruppe, beispielsweise des Windelverschlussbandes, der Befestigungsseite abgewandt.

In vorliegendem Zusammenhang ist der Begriff "hinter" als eine Richtungsangabe in Bezug auf die Befestigungsseite und die Rückseite zu verstehen, indem die Befestigungsseite als vorne liegend definiert wird. Dementsprechend liegt alles, was von einer Baugruppe ausgehend in Richtung auf die Rückseite angeordnet ist, hinter der entsprechenden Baugruppe. So kann beispielsweise die Befestigungsseite einer Trägerbahn als Vorderseite der Trägerbahn angesehen werden, wie auch der Begriff der Rückseite entsprechend die andere, der Befestigungsseite gegenüberliegenden Seite der Trägerbahn bezeichnet. So beschreibt der Begriff "hinter" im Wesentlichen einen Bereich eines applizierten Windelverschlussbandes, welcher von dem Befestigungsgebiet zumindest teilweise überdeckt ist, wodurch klar ist, dass dieser Bereich nicht neben dem Befestigungsgebiet platziert ist, wie auch aus den Darstellungen der nachfolgend erläuterten Ausführungsbeispielen gut ersichtlich ist.

Vorteilhafter Weise kann das vorliegende Windelverschlussband sowohl an einer Babywindel als auch an einer sonstigen Inkontinenzwindel eingesetzt werden. Deshalb sei an dieser Stelle bereits erwähnt, dass die Aufgabe der Erfindung auch von jeglicher Windel mit einem Windelverschlussband nach einem der erläuterten Merkmale gelöst wird.

Ein derartiges Windelverschlussband kann ohne Weiteres mittels eines Halbzeugstreifens bereitgestellt werden, welcher insbesondere eine Trägerbahn umfasst, auf deren Befestigungsseite die Komponente des zweikomponentigen Verschlusssystems angeordnet ist, wobei auf der Rückseite der Trägerbahn eine Klebstoffbeschichtung durchgängig vorgesehen ist. Insofern wird die Aufgabe der Erfindung auch von einem Halbzeugstreifen umfassend eine Trägerbahn mit einer Befestigungsseite und mit einer Rückseite gelöst, bei welchem auf der Befestigungsseite der Trägerbahn eine Komponente eines zweikomponentigen Verschlusssystems angeordnet ist, wobei auf der Rückseite der Trägerbahn eine Klebstoffbeschichtung durchgängig vorgesehen ist.

Mittels dieser Klebstoffbeschichtung kann ein entsprechend konfektionierter Halbzeugstreifen konstruktiv einfach an einer Windel, insbesondere an einem Windelohr, appliziert und hierbei zu dem vorliegenden Windelverschlussband funktionalisiert werden.

Ein aus einem derartigen Halbzeugstreifen geschnittenes Windelverschlussband kann dann insbesondere an einer Windel bzw. um ein Windelohr derart umgefaltet werden, dass durch die Klebstoffbeschichtung die Trägerbahn im entsprechenden Teilgebiet doppelt ausgebildet ist und auch so dauerhaft verbleibt. Dementsprechend kann sich hierdurch auf konstruktiv einfache Weise eine entsprechend angenehmere Haptik an dem Windelverschlussband ausbilden. Bei einer derartigen Vorgehensweise verbleibt insbesondere ein Teil der Windel bzw. ein Teil des Windelohrs zwischen den beiden Lagen der Trägerbahn.

Hierbei ist es nicht zwingend notwendig, dass die Klebstoffbeschichtung hinter dem Befestigungsgebiet vorgesehen ist. Wird hierauf verzichtet, weist das Windelverschlussband in diesem Bereich zwar die gewünschten Verbesserungen in der Haptik auf, ist jedoch ansonsten äußerst weich ausgebildet, so dass die Komponente des zweikomponentigen Verschlusssystems einerseits eine gute Verbindung mit der anderen Komponente des zweikomponentigen Verschlusssystems eingehen kann.

Andererseits kann bei geeigneter Wahl des Trägerbandes, des entsprechenden Klebstoffes, des zweikomponentigen Verschlusssystems und/oder beispielsweise einer Trageschicht für eine mechanische Komponente eines mechanischen Verschlusssystems auch hinter dem Befestigungsgebiet wenigstens teilweise Klebstoff vorgesehen sein, welcher die beiden Lagen der Trägerbahn miteinander verbindet. Hierdurch bedingt kann das Windelverschlussband im Befestigungsgebiet auch etwas versteift werden, was möglicherweise zu etwas geringeren Bindungskräften bei dem zweikomponentigen Verschlusssystem führt. Wird jedoch beispielsweise ein sehr bewegliches Material für die Trägerbahn oder eine Trageschicht eines mechanischen Verschlusssystems gewählt, kann diesem Nachteil entgegen gewirkt werden, so dass ausreichende Bindungskräfte gewährleistet werden können. Hierbei kann der Klebstoff zwei Trägerbahnlagen direkt oder indirekt miteinander verbinden, wobei letzteres beispielsweise durch das Einfassen einer anderen Lage, wie beispielsweise einer durch ein Windelohr gebildeten Lage, erfolgen kann. In diesem Zusammenhang sieht eine vorteilhafte Ausführungsvariante vor, dass hinter dem Befestigungsgebiet wenigstens teilweise Klebstoff vorgesehen ist, der zwei Trägerbahnlagen direkt oder indirekt miteinander verbindet.

Dementsprechend kann sich ein Windelverschlussband mit einer Befestigungsseite und mit einer Rückseite, bei welchem auf der Befestigungsseite in wenigstens einem Befestigungsgebiet eine Komponente eines zweikomponentigen Verschlusssystem vorgesehen ist, die auf einer Trägerbahn angeordnet ist, und bei welchem das Windelverschlussband in einem Permanentbereich an einer Windel zu befestigen ist, dadurch auszeichnen, dass die Trägerbahn zumindest in dem Permanentbereich mehrlagig ausgebildet ist, um ein Windelverschlussband bereitzustellen, welches insbesondere im Bereich eines Befestigungsgebietes eine außergewöhnlich angenehme Haptik gewährleistet und dennoch aus einem möglichst einfach aufgebauten Halbzeug gefertigt werden kann.

Auf diese Weise kann insbesondere im Bereich, in welchem das Windelverschlussband permanent an einer Windel befestigt ist, eine entsprechende, angenehme Haptik realisiert werden. Es versteht sich, dass der Begriff "zu befestigen" sich auf das Windelverschlussband als solches bezieht und, wenn eine Windel mit dem Windelverschlussband im Permanentbereich permanent verbunden ist, den Begriff "befestigt" mit umfasst.

Der Begriff "Permanentbereich" beschreibt vorliegend denjenigen Bereich des Windelverschlussbandes, mittels welchem das Windelverschlussband permanent, also im Wesentlichen unlösbar, an der Windel befestigt ist. Der Permanentbereich unterscheidet sich demnach von dem bereits vorstehend erläuterten Fasteningbereich, welcher eine lösbare Verbindung ermöglicht, so dass eine Windel an- und wieder abgelegt werden kann. Hierbei müssen die beiden Bereiche nicht nebeneinander angeordnet oder sogar über einen bandförmigen Zwischenbereich miteinander verbunden sein, der Permanentbereich kann auch hinter dem Fasteningbereich vorgesehen sein.

Insofern wird vorzugsweise im Permanentbereich ein irreversibles Befestigungsmittel, wie insbesondere eine Schweiß- oder Klebeverbindung, vorgesehen sein, welche entsprechend unter normalen Betriebszuständen eine unlösbare Verbindung mit einer Windel ermöglicht. Im Gegensatz hierzu ist die vorliegende Komponente des zweikomponentigen Verschlusssystems als reversibles Befestigungsmittel anzusehen, welches bei Bedarf auch mehrfach von einem Benutzer einer Windel betätigt werden kann.

Vorzugsweise ist in dem Permanentbereich wenigstens eine Lage der Trägerbahn vor und eine Lage der Trägerbahn hinter der Windel angeordnet. Auf diese Weise kann von einem äußerst einfach aufgebauten Halbzeug ausgehend eine sehr stabile Verbindung zwischen Windelverschlussband und Windel gewährleistet werden. Insbesondere können Abschereffekte, wie beispielsweise der sogenannte "Powerstrip-Effekt", minimiert werden.

Zur Applikation des Windelverschlussbandes an einer Windel braucht, um ein Windelverschlussband bereitzustellen, welches insbesondere im Bereich eines Befestigungsgebietes eine außergewöhnlich angenehme Haptik gewährleistet und dennoch aus einem möglichst einfach aufgebauten Halbzeug gefertigt werden kann, somit das Windelverschlussband mittels eines Applizierverfahrens lediglich von einem entsprechenden Halbzeugstreifen senkrecht zu einer Maschinenrichtung abgetrennt und an der Windel appliziert werden, wobei die Trägerbahn des Windelverschlussbandes, auf welcher die Komponente des zweikomponentigen Verschlusssystem angeordnet ist, umgefaltet und beidseits an der Windel befestigt wird. Die Applikation eines Windelverschlussbandes an einer Windel ist mithin besonders einfach und betriebssicher zu gewährleisten.

Das auf diese Weise bereitgestellte Windelverschlussband umgreift somit ähnlich wie ein Windelverschlussband, welches einen sogenannten Y-Bond aufweist, eine Windel bzw. ein entsprechendes Windelohr, und das vorliegende Windelverschlussband weist einerseits somit alle Vorteile einer derartigen Verbindung auf. Andererseits ist das auf die vorstehend beschriebene Weise bereitgestellte Windelverschlussband und mithin das entsprechende Halbzeug wesentlich einfacher aufgebaut als bekannte gattungsgemäße Windelverschlussbänder.

Des Weiteren wird, unabhängig von den übrigen Merkmalen vorliegender Erfindung ein Windelverschlussband bzw. ein Halbzeugstreifen mit einer Befestigungsseite und mit einer Rückseite vorgeschlagen, wobei auf der Befestigungsseite in wenigstens einem Befestigungsgebiet eine Komponente eines zweikomponentigen Verschlusssystems vorgesehen ist, die auf einer Trägerbahn angeordnet ist, wobei die Trägerbahn auf ihrer Befestigungsseite eine dehäsive Beschichtung aufweist, und wobei sich das Windelverschlussband bzw. der Halbzeugstreifen dadurch auszeichnen, dass Bestandteile der dehäsiven Beschichtung hinter der Komponente des zweikomponentigen Verschlusssystems zu finden sind.

In Abweichung von der herrschenden Meinung, nach welcher eine dehäsive Beschichtung lediglich auf freien Oberflächenbereichen vorgesehen sein sollte, wo diese auch wirksam ist, und nach welcher im Übrigen eine derartige dehäsive Beschichtung gerade in abgedeckten Bereichen nicht vorgesehen sein sollte, da diese dehäsive Beschichtung etwaige Bindungskräfte minimiert bzw. minimieren könnte, schlägt somit vorliegende Erfindung gerade vor, eine derartige Beschichtung auch hinter einer Komponente des zweikomponentigen Verschlusssystems vorzusehen. Eine derartige Vorgehensweise ermöglicht eine besonders einfache Herstellung des Windelverschlussbandes bzw. des entsprechenden Halbzeugs, da die dehäsive Beschichtung nicht mehr so genau positioniert werden muss. Darüber hinaus können unschöne Übergänge und insbesondere auch haptische Beeinträchtigungen durch derartige Übergänge vorteilhafter Weise vermieden werden.

Insbesondere ist es möglich, die dehäsive Beschichtung durchgängig auch hinter der Komponente des zweikomponentigen Verschlusssystems vorzusehen, so dass bei der Herstellung des Windelverschlussbandes bzw. des entsprechenden Halbzeugstreifens eine passgenaue Positionierung der dehäsiven Beschichtung insbesondere hinsichtlich der Komponente des zweikomponentigen Verschlusssystems nicht weiter erforderlich ist. Es ist somit insbesondere sogar möglich, ein bereits mit einer entsprechenden dehäsiven Beschichtung vollflächig versehenes Band zuzukaufen, ohne dass dem jeweiligen Hersteller bezüglich einer dehäsiven Beschichtung Vorgaben über deren Lage gemacht werden müssen. Insbesondere können aus breiteren Streifen derartiger Bänder entsprechende Teilbänder im Nachhinein geschnitten werden. Beispielsweise ist eine streifenförmige und/oder Silikonisierung des Trägerbandes des Windelverschlussbandes nicht mehr erforderlich, so dass die Herstellung des Windelverschlussbandes vereinfacht werden kann.

In diesem Zusammenhang ist es jedoch entsprechend bereits vorteilhaft, wenn die dehäsive Beschichtung bis hinter die Komponente des zweikomponentigen Verschlusssystems reicht.

Um je nach konkreten Verhältnissen eine ausreichende Verbindung zwischen der Komponente des zweikomponentigen Verschlusssystems und dem Trägerband über eine Klebstoffschicht zu gewährleisten, wenn die Klebstoffschicht wenigstens teilweise auf die dehäsive Beschichtung aufgebracht ist, kann es vorteilhaft sein, die Klebstoffschicht unter zusätzlichem Energieeintrag mit der Trägerbahn zu verbinden.

Deshalb wird die Erfindung auch von einem Verfahren zum Herstellen eines Halbzeugstreifens mit einer Befestigungsseite und mit einer Rückseite gelöst, wobei auf der Befestigungsseite in wenigstens einem Befestigungsgebiet eine Komponente eines zweikomponentigen Verschlusssystems vorgesehen ist, die über eine Klebstoffschicht mit einer Trägerbahn, welche auf der Befestigungsseite mit einer dehäsiven Beschichtung versehen ist, verbunden ist, bei welchem die Klebstoffschicht wenigstens teilweise auf die dehäsive Beschichtung aufgebracht und unter zusätzlichem Energieeintrag mit der Trägerbahn verbunden wird.

Der zusätzliche Energieeintrag gewährleistet trotz der dehäsiven Beschichtung eine ausreichende Verbindungsstärke.

Insbesondere kann der zusätzliche Energieeintrag beispielsweise dadurch vorgesehen sein, dass der Klebstoff in verformbarem, flüssigem und/oder heißem Zustand mit der Trägerbahn verbunden wird. Auf diese Weise kann eine besonders innige Verbindung erfolgen, welche der dehäsiven Wirkung der dehäsiven Beschichtung entgegenwirkt. Insbesondere bei einer textilen Trägerbahn kann die Klebstoffschicht in Zwischenräume der textilen Klebstoffschicht eindringen. Letztere ist nicht zwingend notwendig, wenn bei einer textilen Trägerbahn lediglich Teile der Fasern die entsprechende Beschichtung tragen, während im Übrigen noch ohne Weiteres der Klebstoff an den Fasern angreifen kann.

Durch den zusätzlichen Energieeintrag, insbesondere beispielsweise auch durch ein Verpressen, kann gewährleistet werden, dass der Klebstoff möglichst viele der nicht beschichteten Fasern erreicht. Insbesondere bei der Verwendung eines heißen oder warmen Klebstoffes kann die thermische Energie noch zu einem lokalen Lösen oder Auflösen der dehäsiven Beschichtung führen, was wiederum zu einer guten Verbindung führt, wobei in der Regel dann noch Bestandteile der dehäsiven Beschichtung in dem Klebstoff bzw. sonst wo unter der Komponente des mechanischen Verschlusssystems zu finden sein werden.

Die Verbindungsintensität kann zusätzlich oder alternativ erhöht werden, wenn die Klebstoffschicht mit der Trägerbahn verpresst wird.

Ggf. wird insbesondere bei einem hohen Eintrag an thermischer Energie die dehäsive Beschichtung zur Gänze umgesetzt, indem sie beispielsweise verdampft wird. Bei einer derartigen Verfahrensführung wird dann eine entsprechend innige Verbindung zwischen der Klebstoffschicht und der Trägerbahn bzw. zwischen der Komponente des mechanischen Verschlusssystems und der Trägerbahn gewährleistet, wobei dann die dehäsive Beschichtung bis unmittelbar an die Klebstoffschicht heranreicht. Bei aus dem Stand der Technik bekannten Windelverschlussbändern hingegen verbleibt in der Regel ein Abstand zwischen der dehäsiven Beschichtung und der Klebstoffschicht, der durch Fertigungstoleranzen bedingt ist.

Als entsprechende Komponente des mechanischen Verschlusssystems kann einerseits die vorstehend erläuterte Klebstoffschicht unmittelbar zur Anwendung kommen, wenn es sich um ein klebend wirksames Verschlusssystem handelt. Andererseits kann diese Klebstoffschicht auch zur Befestigung einer Trägerschicht für die Komponente des mechanischen Verschlusssystems, insbesondere für eine Komponente eines mechanischen Verschlusssystems, dienen.

Eine bevorzugte Ausführungsvariante sowohl hinsichtlich des Windelverschlussbandes als auch hinsichtlich des Halbzeugstreifens sieht vor, dass die Trägerbahn eine textile Trägerbahn ist und die Komponente des zweikomponentigen Verschlusssystems über eine Klebstoffschicht mit der textilen Trägerbahn verbunden und die Klebstoffschicht in Zwischenräume der textilen Trägerbahn eingedrungen ist. Bei einer textilen Trägerbahn kann die Klebstoffschicht zumindest teilweise in Zwischenräume der textilen Trägerbahn eindringen, so dass die Komponente des zweikomponentigen Verschlusssystems eine besonders innige Verbindung mit vorliegender Trägerbahn eingehen kann.

In einer vorteilhaften Ausführungsvariante des Halbzeugstreifens kann dieser zwei voneinander beabstandete Bereiche jeweils eine Komponente des zweikomponentigen Verschlusssystems tragen, wobei mittig zwischen diesen Bereichen eine Symmetrieebene liegt, bezüglich welcher der Halbzeugstreifen symmetrisch ausgebildet ist. Mittels eines derart ausgebildeten Halbzeugstreifens können insbesondere die vorliegenden Windelverschlussbänder konstruktiv besonders einfach bereitgestellt werden, da häufig je Windel genau zwei Windelverschlussbänder benötigt werden, die dann mittels Durchtrennen des Halbzeigstreifens in seiner Symmetrieebene bereit gestellt werden können.

Vorzugsweise ist die Komponente des zweikomponentigen Verschlusssystems eine Komponente eines mechanischen Verschlusssystems, da bei derartigen mechanischen Verschlusssystemen eine stabile Anbindung der jeweiligen Komponente an die Windel erstrebenswert ist. Es versteht sich, dass ein solches mechanisches Verschlusssystem vielfältig ausgebildet sein kann, insbesondere hinsichtlich seiner Verschluss-Komponenten. Vorteilhaft ist es insbesondere unter der Maßgabe, ein möglichst günstiges Verschlusssystem bereitzustellen, wenn die Komponente des mechanischen Verschlusssystems, welche an dem Windelverschlussband befestigt ist, die Hakenkomponente eines Haken-Schlaufen-Verschlusssystems ist. Haken-Schlaufen-Verschlusssysteme haben sich vielfach als besonders sichere und einfach zu bedienende Verschlusssysteme bewährt.

Bei einer weiter bevorzugten Ausführungsvariante handelt es sich bei dem Haken-Schlaufen-Verschlusssystem um eine Komponente eines Mikrokletts, welches als eine erste Komponente des mechanischen Verschlusssystems auf der Trägerbahn des Windelverschlussbandes beziehungsweise des Halbzeugstreifens in geeigneter Weise aufgeklebt ist.

Ist die Trägerbahn einstückig ausgebildet und an einer Knicklinie umgefaltet, kann das Windelverschlussband besonders einfach an einer Windel appliziert werden.

Kumulativ zu dem vorstehend erläuterten Applikationsverfahren kann es vorteilhaft sein, wenn vor, während oder nach dem Umfalten die Trägerbahn durchtrennt wird. Auf diese Weise kann einfach und betriebssicher auf eine Knickstelle bzw. eine Faltlinie verzichtet werden, welche zu ggf. zu einer unerwünschten Versteifung führen könnte. Dieses gilt insbesondere dann, wenn die Trägerbahn nach einer Applikation an einer Windel oder an einem Windelohr einen Rand der Windel bzw. des Windelohrs umgreift. Alternativ kann jedoch auch ohne Weiteres gerade eine derartige Knickstelle bzw. Faltlinie erwünscht sein, um auch im Bereich des vorgenannten Randes eine entsprechende Stabilisierung zu gewährleisten.

Insbesondere auch unter Berücksichtigung der in vorstehendem Absatz aufgeführten Überlegungen kann die Bereitstellung eines Windelverschlussbandes vereinfacht werden, wenn zwei der Lagen der Trägerbahn identisch aber bezüglich einer zwischen den Lagen liegenden Ebene spiegelsymmetrisch ausgebildet sind. Diese kann beispielsweise durch einen Umfaltprozess einfach und betriebssicher dargestellt werden.

Es versteht sich, dass der vorstehend beschriebene Halbzeugstreifen als Halbzeugrolle aufgerollt, als Halbzeugspule aufgespult oder als Halbzeugwickelung aufgewickelt bzw. in Schleifen gelegt zur Anwendung kommen können. Insbesondere in den vorgenannten Gebinden kann ein Transport oder Versand einfach und betriebssicher gewährleistet werden.

Weitere Vorteile, Ziele und Eigenschaften vorliegender Erfindung werden anhand der Beschreibung anliegender Zeichnung beschrieben, in welcher beispielhaft unterschiedlich ausgebildete Halbzeugstreifen und hieraus hergestellte Windelverschlussbänder, die an nahezu beliebigen Windeln appliziert sein können, dargestellt sind. Komponenten, welche jeweils in einem Ausführungsbeispiel der Figuren wenigstens im Wesentlichen hinsichtlich ihrer Funktionen übereinstimmen, können hierbei mit gleichen Bezugsziffern bezeichnet sein, wobei diese Komponenten nicht in allen Figuren beziffert und erläutert sein müssen. Es zeigt
- Figur 1: schematisch einen ersten erfindungsgemäßen, zwei-nutzigen Halbzeugstreifen mit zwei voneinander beabstandeten Bereichen, die jeweils eine Komponente eines zweikomponentigen Verschlusssystems tragen;
- Figur 2: schematisch eine Ansicht einer ersten Befestigungsvariante eines aus dem ersten Halbzeugstreifen aus der Figur 1 hergestellten Windelverschlussbandes;
- Figur 3: schematisch eine Ansicht einer weiteren Befestigungsvariante eines aus dem ersten Halbzeugstreifen aus der Figur 1 hergestellten Windelverschlussbandes;
- Figur 4: schematisch eine Ansicht einer weiteren Befestigungsvariante des aus dem ersten Halbzeugstreifen aus der Figur 1 hergestellten Windelverschlussbandes;
- Figur 5: schematisch eine Ansicht eines ähnlichen Windelverschlussbandes, bei welchem die Komponente des zweikomponentigen Verschlusssystems weiter entfernt vom Ende einer Trägerbahn des Windelschlussbandes als bei den Ausführungsbeispielen nach Figuren 1 bis 4 angeordnet ist;
- Figur 6: schematisch eine Ansicht eines ähnlichen Windelverschlussbandes mit einer gegenüber dem Ausführungsbeispiel nach Figur 5 kleineren Komponente des zweikomponentigen Verschlusssystems, bei welcher die kleinere Komponente ebenfalls weiter entfernt vom Ende der Trägerbahn des Windelschlussbandes angeordnet ist;
- Figur 7: schematisch einen zweiten zu einem erfindungsgemäßen Windelverschlussband trennbaren zwei-nutzigen Halbzeugstreifen, der ähnlich dem Halbzeugstreifen nach Figur 1 ausgebildet ist, mit einer teilweise ausgesparten Klebstoffbeschichtung;
- Figur 8: schematisch eine Ansicht einer Befestigungsvariante eines aus dem zweiten Halbzeugstreifen hergestellten Windelverschlussbandes mit der teilweise ausgesparten Klebstoffbeschichtung hinter einer Komponente eines zweikomponentigen Verschlusssystems;
- Figur 9: schematisch einen dritten erfindungsgemäßen, zwei-nutzigen Halbzeugstreifen, der ähnlich des Halbzeugstreifen nach Figur 1 ausgebildet ist, mit einer alternativen Komponente eines zweikomponentigen Verschlusssystems;
- Figur 10: schematisch eine Ansicht einer ersten Befestigungsvariante eines aus dem dritten Halbzeugstreifen aus der Figur 9 hergestellten Windelverschlussbandes;
- Figur 11: schematisch eine Ansicht einer alternativen Befestigungsvariante des aus dem dritten Halbzeugstreifen aus der Figur 9 hergestellten Windelverschlussbandes;
- Figur 12: schematisch eine Ansicht einer weiteren Befestigungsvariante des aus dem dritten Halbzeugstreifen aus der Figur 9 hergestellten Windelverschlussbandes;
- Figur 13: schematisch eine Ansicht eines ähnlichen Windelverschlussbandes, bei welcher die alternative Komponente des zweikomponentigen Verschlusssystems weiter entfernt vom Ende der Trägerbahn angeordnet ist;
- Figur 14: einen vierten zu einem erfindungsgemäßen Windelverschlussband trennbaren, zwei-nutzigen Halbzeugstreifen, der ähnlich dem Halbzeugstreifen nach Figur 7 ausgebildet ist;
- Figur 15: schematisch eine Ansicht einer Befestigungsvariante eines aus dem vierten Halbzeugstreifen aus der Figur 14 hergestellten Windelverschlussbandes;
- Figur 16: einen fünften zu einem erfindungsgemäßen Windelverschlussband trennbaren, zwei-nutzigen Halbzeugstreifen, der ähnlich dem Halbzeugstreifen nach Figur 14 ausgebildet ist; und
- Figur 17: schematisch eine Ansicht einer Befestigungsvariante eines aus dem fünften Halbzeugstreifen nach Figur 16 hergestellten Windelverschlussbandes.

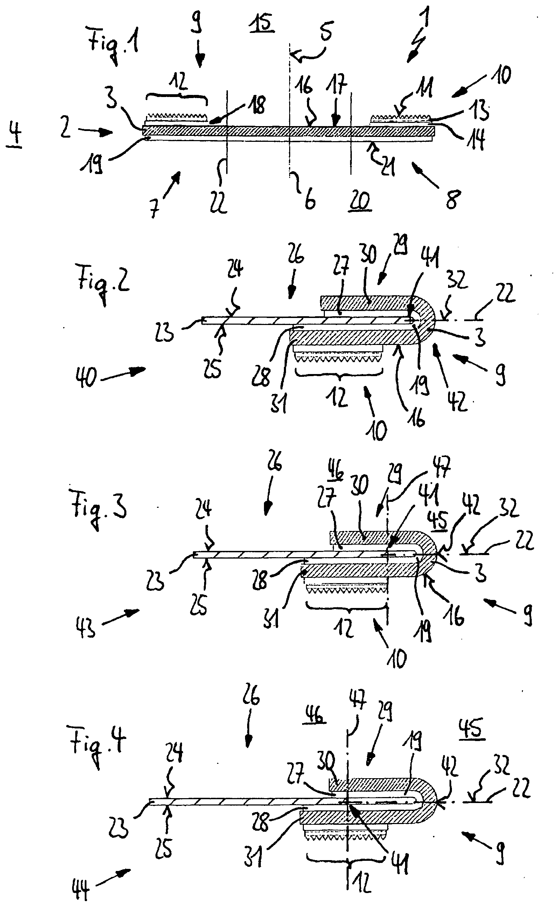

Der in der Figur 1 gezeigte erste Halbzeugstreifen 1 ist als Trägerband 2 ausgebildet und erstreckt sich als Trägerbahn 3 senkrecht zur Papierebene 4.

Der Halbzeugstreifen 1 ist hinsichtlich einer Symmetrieebene 5 symmetrisch ausgebildet, wobei die Symmetrieebene 5 gleichzeitig eine Trennlinie 6 zweier Halbzeugstreifennutzen 7 und 8 markiert. Insofern kann mit jedem der Halbzeugstreifennutzen 7 und 8 jeweils ein Windelverschlussband 9 (hier nur exemplarisch beziffert) bereitgestellt werden.

Neben der Trägerbahn 3 umfasst der Halbzeugstreifen 1 eine Komponente 10 eines zweikomponentigen mechanische Verschlusssystems, welche in diesem Ausführungsbeispiel als Haken 11 eines Haken-Schlaufen-Verschlusssystems (hier nicht vollständig dargestellt) ausgebildet ist. Die Komponente 10 bildet hierbei ein Befestigungsgebiet 12 an dem Windelverschlussband 9. Die Komponente 10 umfasst des Weiteren eine Trägerschicht 13 für die Haken 11, wobei die Trägerschicht 13 mittels einer Klebstoffschicht 14 auf die Trägerbahn 3 aufgeklebt ist.

Der Halbzeugstreifen 1 ist bei diesem Ausführungsbeispiel an seiner Befestigungsseite 15 vollständig mit einer dehäsiven Beschichtung 16 versehen, welche zumindest in diesem Ausführungsbeispiel durch eine Silikonschicht 17 realisiert ist. Hierbei befinden sich zumindest Bestandteile der dehäsiven Beschichtung 16 auch hinter der Komponente 10 des zweikomponentigen mechanischen Verschlusssystems, wodurch die Klebstoffschicht 14 im Wesentlichen direkt auf die Silikonschicht 17 der Trägerbahn 3 aufgetragen ist. Da die Trägerbahn 3 hier jedoch vorteilhafter Weise aus einem textilen Trägerbahnmaterial hergestellt ist, kann sich die Klebstoffschicht 14 zumindest teilweise in Zwischenräume (hier nicht dargestellt) des textilen Trägerbahnmaterials (nicht gesondert beziffert) eindringen, so dass mittels der Klebstoffschicht 14 auch eine gute Formschlussverbindung zwischen der Komponente 10, insbesondere zwischen der Trägerschicht 13, und der Trägerbahn 3 realisiert ist. Somit kann die Komponente 10 trotz der dehäsiven Beschichtung 16 der Trägerbahn 3 eine besonders innige Verbindung zu der Trägerbahn 3 herstellen.

Der Halbzeugstreifen 1 weist neben der Klebstoffschicht 14, welche hinsichtlich der Befestigungsseite 15 einen ersten Klebstoffauftrag 18 insbesondere an dem Windelverschlussband 9 bildet, einen zweiten Klebstoffauftrag 19 auf, welcher an einer Rückseite 20 des Halbzeugstreifens 1 vorgesehen ist.

Insbesondere der zweite Klebstoffauftrag 19 bildet an der Rückseite 20 des Halbzeugstreifens 1 einen Permanentbereich 21 aus, mittels welchem später das Windelverschlussband 9 permanent, also dauerhaft, befestigt werden kann. Der zweite Klebstoffauftrag 19 befindet sich bei diesem ersten Ausführungsbeispiel im Wesentlichen durchgängig an der Rückseite 20.

Jeder der Halbzeugstreifennutzen 7 oder 8, welche jeweils ein Windelverschlussband 9 bereitstellen können, weist eine Knicklinie 22 (hier nur exemplarisch beziffert) auf, an welcher das Windelverschlussband 9 beim Applizieren des Windelverschlussbandes 9, beispielsweise an einer Windel 23 (siehe insbesondere Figuren 2 bis 6), eingeknickt bzw. umgeschlagen wird, so dass das Windelverschlussband 9 mittels des zweiten Klebstoffauftrags 19 mit seinem Permanentbereich 21 sowohl an einer Oberseite 24 der Windel 23 als auch an einer Unterseite 25 der Windel 23 angeklebt werden kann. Die Komponente 10 liegt hierbei niemals auf der Knicklinie 22, so dass die Komponente 10 beim Applizieren des Windelverschlussbandes 9 vorteilhafter Weise nie geknickt wird.

Insbesondere nach den Darstellungen der Figuren 2 bis 6 handelt es sich bei der Windel 23 jeweils um ein Windelohr 26 einer Babywindel (hier nicht explizit gezeigt), an welchem beispielhaft der zweite Halbzeugstreifennutzen 8 bereits appliziert worden ist, indem das Windelverschlussband 9 von dem Halbzeugstreifen 1 senkrecht zu einer Maschinenrichtung abgetrennt wurde und die Trägerbahn 3 anschließend mit dem zweiten Klebstoffauftrag 19 an das Windelohr 26 angeklebt, die Trägerbahn 3 im Bereich der Knicklinie 22 umgefaltet und hierbei die Trägerbahn 3 beidseits an der Windel 23 befestigt wurde. Vor dem Applizieren wurde die Trägerbahn 3 also an der Trennlinie 6 durchgetrennt, so dass aus einem einzigen Halbzeugstreifen 1 zwei identische Windelverschlussbänder 9 gewonnen wurden.

Derart appliziert ist das Windelverschlussband 9 hinsichtlich des zweiten Klebstoffauftrags 19 mit einer ersten Klebstofflage 27 und mit einer zweiten Klebstofflage 28 an der Windel 23 angeklebt. Sowohl die erste Klebstofflage 27 als auch die zweite Klebstofflage 28 befinden sich hierbei zumindest teilweise hinter der Komponente 10 des zweikomponentigen mechanischen Verschlusssystems, wodurch sich am Windelverschlussband 9 eine besonders gute Haptik für einen Benutzer einstellt.

Derart appliziert ist insbesondere die Trägerbahn 3 zumindest hinter einem Teilgebiet 29 des Befestigungsgebietes 12 mehrlagig ausgebildet. Insofern ist hinter dem Befestigungsgebiet 12 wenigstens teilweise Klebstoff vorgesehen, der zwei Trägerbahnlagen 30 und 31 direkt, siehe insbesondere die Ausführungsbeispiele nach Figuren 4 bis 6, oder indirekt, siehe insbesondere das Ausführungsbeispiel nach Figur 2 über da Windelohr, miteinander zu zwei Lagen verbindet. Dementsprechend ist auch der Permanentbereich 21 insbesondere hinsichtlich der Klebstofflagen 27 und 28 mehrlagig ausgebildet, wodurch in dem Permanentbereich 21 wenigstens eine erste Trägerbahnlage 30 vor und eine Trägerbahnlagen 31 hinter der Windel 23 angeordnet ist.

Dadurch, dass die Trägerbahn 3 hierbei einstückig ausgebildet und an der Knicklinie 22 umgefaltet ist, ist das Windelverschlussband 9 und damit auch das gesamte Windelohr 26 besonders reißfest ausgebildet. Auch sind die beiden Trägerbahnlagen 30, 31 identisch aber bezüglich einer zwischen den Trägerbahnlagen 30 beziehungsweise 31 liegenden Ebene 32 spiegelsymmetrisch ausgebildet.

Des Weiteren ist unmittelbar nachzuvollziehen, dass Bestandteile der dehäsiven Beschichtung 16 hinter der Komponente 10 des zweikomponentigen mechanischen Verschlusssystems zu finden sind, und somit die dehäsive Beschichtung 16 bis hinter die Komponente 10 des zweikomponentigen mechanischen Verschlusssystems reicht.

Während hinsichtlich der Darstellung nach der Figur 2 eine erste Befestigungsvariante dargestellt ist, bei welcher das Windelverschlussband 9 unmittelbar am Ende 41 des Windelohrs 26 umgefaltet ist und somit ein Scheitelbereich 42 des Windelverschlussbandes 9 relativ nahe am Ende 41 des Windelohrs 26 angeordnet ist, sind hinsichtlich einer zweiten Befestigungsvariante 43 (siehe Figur 3) und einer dritten Befestigungsvariante 44 (siehe Figur 4) jeweils der Scheitelbereich 42 weiter entfernt von dem Ende 41 des Windelohrs 26 angeordnet, wodurch jeweils zusätzlich ein Benutzerbereich 45 des jeweiligen Windelverschlussbandes 9 geschaffen bzw. vergrößert werden kann.

Ein derartiger Benutzerbereich 45 ist jedoch nicht zwingend an einem Windelverschlussband 9 erforderlich. Der Benutzerbereich 45 unterscheidet sich vorliegend von einem Befestigungsbereich 46 dadurch, dass er an einem Windelverschlussband 9 ab einer Grenze 47 beginnt, welche durch das Ende 41 des Windelohrs 46 definiert sein kann. Sofern der Benutzerbereich 45 vorhanden ist, zeichnet er sich zusätzlich immer dadurch aus, dass in seinem Bereich die Trägerbahn 3 doppellagig an dem Windelverschlussband 9 ausgebildet ist. Der Befestigungsbereich 46 hingegen kann sich von dem Benutzerbereich 45 dadurch abheben, dass die Trägerbahn 3 lediglich teilweise doppellagig ausgebildet ist.

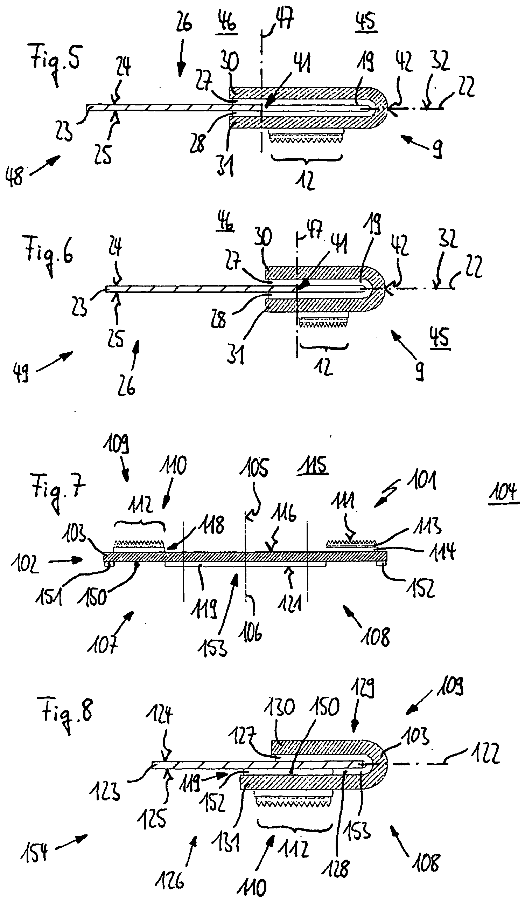

Je nach Befestigungsvariante kann die Komponente 10 des zweikomponentigen mechanischen Verschlusssystems entweder vollflächig im Benutzerbereich 45 (siehe beispielsweise Figur 6) oder vollflächig im Befestigungsbereich 46 (siehe beispielsweise Figur 3) angeordnet sein. Nach der dritten Befestigungsvariante 44 befindet sich die Komponente 10 sowohl teilweise im Benutzerbereich 45 als auch teilweise im Befestigungsbereich 46.

Zudem kleben die beiden Klebstofflagen 27 und 28 im Benutzerbereich 45 in der Regel unmittelbar zusammen und nicht nur mittelbar, wie im Befestigungsbereich 46, da im Befestigungsbereich 46 das Windelohr 26 vorgesehen ist, so dass das Windelverschlussband 9 im Benutzerbereich 45 weniger steif ausgebildet sein kann. Insofern wird im befestigten Zustand des Windelverschlussbandes 9 an der Windel 23 immer ein Befestigungsbereich 46 erzeugt aber nicht immer ein Benutzerbereich 45.

Hinsichtlich einer vierten Befestigungsvariante 48 gemäß der Darstellung nach der Figur 5 befindet sich die Komponente 10 ebenfalls vollständig in dem Benutzerbereich 45, wodurch die Komponente 10 hinsichtlich ihrer Biegefahigkeit flexibler gestaltet werden kann. Hierdurch kann sich auch die Haftfähigkeit der Komponente 10 erhöhen.

Die in der Figur 6 gezeigte fünfte Befestigungsvariante 49 des Windelverschlussbandes 9 zeigt einen kürzer ausgebildeten Benutzerbereich 45, in welchem zudem die Komponente 10 unmittelbar an der Grenze 47 zwischen einem Befestigungsbereich 46 und einem Benutzerbereich 45 platziert ist.

Der in der Figur 7 gezeigte zweite Halbzeugstreifen 101 weist im Wesentlichen einen ähnlichen Aufbau auf, wie der erste Habzeugstreifen 1. So umfasst der zweite Halbzeugstreifen 101 ein Trägerband 102, welches senkrecht zur Papierebene 104 ausgerichtet ist, so dass der zweite Halbzeugstreifen 101 eine Trägerbahn 103 ausbildet. Der zweite Halbzeugstreifen 101 ist hinsichtlich einer Symmetrieebene 105 symmetrisch ausgebildet, so dass er hinsichtlich einer Trennlinie 106 einen ersten Halbzeugstreifennutzen 107 und einen zweiten Halbzeugstreifennutzen 108 ausbildet. Diese Halbzeugstreifennutzen 107 bzw. 108 können jeweils ein Windelverschlussband 109 bereitstellen, wenn sie in geeigneter Weise von dem Trägerband 2 abgetrennt werden.

Das Windelverschlussband 109 umfasst eine Komponente 110 eines zweikomponentigen mechanischen Verschlusssystems, wobei auch hier die Komponente 110 Haken 111 umfasst und somit ein Befestigungsgebiet 112 an dem Windelverschlussband 109 definiert. Die Komponente 110 umfasst eine Trägerschicht 113 und ist mittels einer Klebstoffschicht 114 an einer Befestigungsseite 115 auf einer dehäsiven Beschichtung 116 an der Trägerbahn 103 angeklebt. Die Klebstoffschicht 114 bildet einen ersten Klebstoffauftrag 118, während ein zweiter Klebstoffauftrag 119 an der Rückseite 120 des zweiten Halbzeugstreifens 101 angeordnet ist, der einen Permanentbereich 121 ausbildet.

Der zweite Klebstoffauftrag 119 beschränkt sich jedoch auf Bereiche an der Rückseite 120 des Halbzeugstreifens 101, in welchen an der Befestigungsseite 115 keine Komponenten 110 des zweikomponentigen mechanischen Verschlusssystems vorgesehen sind, so dass hinter den Komponenten 110 hinsichtlich des Permanentbereiches 121 Klebstoffaussparungen 150 (hier nur exemplarisch beziffert) vorgesehen sind. So bildet der zweite Klebstoffauftrag 119 hinsichtlich des Permanentbereiches 121 einen ersten seitlichen Klebstoffauftragsstreifen 151, einen zweiten seitlichen Klebstoffauftragsstreifen 152 und einen mittleren Klebstoffauftragsstreifen 153 aus.

Wird nun der zweite Halbzeugstreifennutzen 108 als Windelverschlussband 109 an einem Windelohr 126 einer Windel 123 und an einer Knicklinie 22 umgefaltet, so dass der Permanentbereich 121 mit einer ersten Klebstofflage 127 an einer Oberseite 124 der Windel 123 und mit einer zweiten Klebstofflage 128 an einer Unterseite 125 der Windel 123 angeklebt ist, befindet sich hinter der Komponente 110 lediglich die erste Klebstofflage 127 des zweiten Klebstoffauftrags 119, so dass die Komponente 110 und damit auch die Haken 111 des Befestigungsgebietes 112 elastischer an dem Windelverschlussband 9 gelagert sind, als dies der Fall wäre, wenn hinter der Komponente 110 neben der ersten Klebstofflage 127 auch die zweite Klebstofflage 128 angeordnet wäre.

Ansonsten ist die in der Figur 8 gezeigte Befestigungsvariante 154 des alternativen Windelverschlussbandes 109 im Wesentlichen identisch mit der ersten Befestigungsvariante 40 des Windelverschlussbandes 9 des ersten Halbzeugstreifens 1, so dass auch bei der Befestigungsvariante 154 eine erste Trägerbahnlage 130 und eine zweite Trägerbahnlage 131 der Trägerbahn 103 hinter der Komponente 110 angeordnet sind, wodurch die Trägerbahn 103 zumindest hinter einem Teilgebiet 129 des Befestigungsgebietes 112 mehrlagig ausgebildet ist.

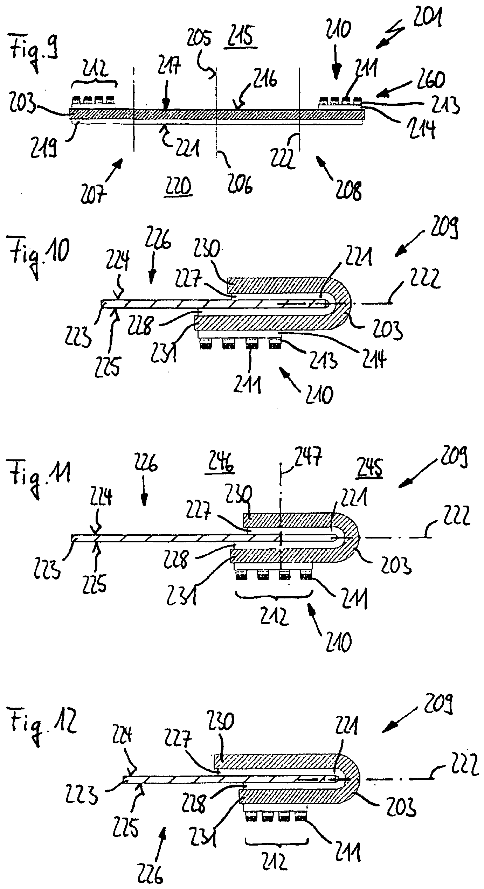

Der in der Figur 9 gezeigte dritte Halbzeugstreifen 201 ist hinsichtlich einer Symmetrieebene 105 symmetrisch ausgebildet und umfasst einen ersten Halbzeugstreifennutzen 207 und einen zweiten Halbzeugstreifennutzen 208, welche beide an einer Trennlinie 206 getrennt jeweils ein Windelverschlussband 209 bereitstellen können. Jedes der Windelverschlussbänder 209 weist dann eine Komponente 210 mit Haken 211 auf, die ein Befestigungsgebiet 212 auf einer Befestigungsseite 215 des jeweiligen Windelverschlussbandes 209 bilden. Die Komponente 210 ist bei diesem Ausführungsbeispiel mit Mikroklettsegmenten 260 ausgestattet, die mit einer Trägerschicht 213 und einer Klebstoffschicht 214 auf einer dehäsiven Beschichtung 216 in Gestalt einer Silikonschicht 217 der Trägerbahn 203 aufgeklebt sind, wobei die Klebstoffschicht 214 durchgehend ausgebildet ist, so dass sie in den Zwischenbereichen zwischen den Mikroklettsegmenten 260 auch klebend wirksam werden kann, wenn das zweikomponentige Verschlusssystem geschlossen wird..

An einer Rückseite 220 des dritten Halbzeugstreifens 201 befindet sich ein Permanentbereich 121, mittels welchem das jeweilige Windelverschlussband 209 an einer Windel 223 (siehe Figuren 10 bis 13) appliziert werden kann. Hierzu umfasst der Permanentbereich 221 einen zweiten Klebstoffauftrag 219.

In den Darstellungen der Figuren 10 bis 13 sind hinsichtlich der Windelverschlussbänder 209 nach Art des dritten Halbzeugstreifens 201 verschiedene Befestigungsvarianten illustriert.

Nach Figur 10 kann das Windelverschlussband 9 derart an einer Knicklinie 222 umgefaltet und an der Windel 223 angeklebt, dass sich sowohl eine erste Klebstofflage 227 des Permanentbereiches 221 als auch eine erste Trägerbahnlage 230 nur teilweise hinter der Komponente 210 befinden, während sowohl eine zweite Klebstofflage 228 des Permanentbereiches 221 als auch eine zweite Trägerbahnlage 231 der Trägerbahn 203 vollständig hinter der Komponente 210 angeordnet sind. Jedenfalls ist der Permanentbereich 221 beidseits der Windel 223 bzw. sowohl an einer Oberseite 224 als auch an einer Unterseite 225 der Windel 223 bzw. eines Windelohrs 226 der Windel 223 befestigt.

Wie in Figur 11 exemplarisch dargestellt, kann das Windelverschlussband 209 derart an dem Windelohr 226 appliziert werden, dass am Windelverschlussband 209 hinsichtlich einer Grenze 247 ein Benutzerbereich 245 und ein Befestigungsbereich 246 gut ausgebildet sind. Insbesondere ist die Komponente 210 bei diesem Ausführungsbeispiel derart an dem Windelverschlussband 209 vorgesehen, dass sich die Komponente 210 sowohl teilweise in dem Benutzerbereich 245 als auch teilweise in dem Befestigungsbereich 246 erstreckt. Während hierbei eine erste Trägerbahnlage 230 und eine zweite Trägerbahnlage 231 befestigungsbereichseitig nur mittelbar mittels der ersten Klebstofflage 227 und der zweiten Klebstofflage 228 miteinander verbunden sind, sind die erste Trägerbahnlage 230 und die zweite Trägerbahnlage 231 benutzerbereichsseitig mittels der beiden Klebstofflagen 227 und 228 unmittelbar miteinander verklebt, da der Benutzerbereich 245 keine Baugruppe der restlichen Windel aufweist beziehungsweise weil dort kein Windelohr 226 vorgesehen ist. Hierdurch ist der Benutzerbereich 245 ein wenig elastischer als der Befestigungsbereich 246 ausgebildet, in welchem die Windel 223 zwischen den beiden Trägerbahnlagen 230 und 231 angeordnet ist.

Gemäß der Darstellung nach der Figur 12 kann das Windelverschlussband 209 des dritten Halbzeugstreifens 201 derart an der Windel 223 appliziert werden, dass einerseits keine wesentliche Unterscheidung zwischen einem Benutzerbereich 245 und einem Befestigungsbereich 246 vorgenommen werden kann bzw. braucht, wie noch hinsichtlich der Darstellung nach Figur 11 erläutert. Andererseits befindet sich hinter der Komponente 210 hinsichtlich des Permanentbereiches 221 sowohl eine erste Klebstofflage 227 des zweiten Klebstoffauftrags 219 als auch eine zweite Klebstofflage 228 des zweiten Klebstoffauftrags 219. Auch ist eine erste Trägerbahnlage 230 und eine zweite Trägerbahnlage 231 der Trägerbahn 203 vollständig hinter der Komponente 210 angeordnet, so dass das Windelverschlussband 209 insbesondere in seinem Befestigungsgebiet 212 relativ fest und starr ausgebildet werden kann.

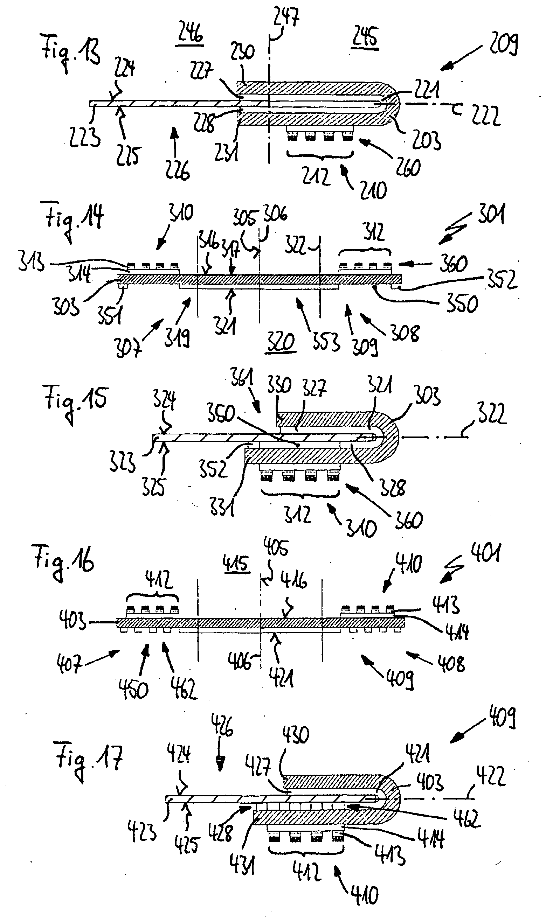

Bei der Darstellung nach der Figur 13 ist ein Windelverschlussband 209 derart an der Windel 223 appliziert, dass sowohl die erste Klebstofflage 227 als auch die zweite Klebstofflage 228 und auch die erste Trägerbahnlage 230 und die zweite Trägerbahnlage 231 symmetrisch beidseits des Windelohrs 226 an der Oberseite 224 bzw. an der Unterseite 225 der Windel 223 appliziert ist. Hierbei ist die Komponente 210 vollständig in dem Benutzerbereich 245 des Windelverschlussbandes 209 platziert, so dass die Komponente 210 beweglicher an der Windel 223 angeordnet werden kann, da im Benutzerbereich 245 die Windel 223 als Zwischenlage fehlt.

Bei dem vierten zweinutzigen Halbzeugstreifen 301 (siehe Figur 14) ist hinter einer jeweiligen Komponente 310 eines ersten Halbzeugstreifennutzens 307 bzw. eines zweiten Halbzeugstreifennutzens 308 eine Klebstoffaussparung 350 (hier nur exemplarisch beziffert) vorgesehen, ähnlich wie bei dem zweiten Halbzeugstreifen 101 bereits beschrieben. Vorliegend ist die Komponente 310 des zweitkomponentigen mechanischen Verschlusssystems jedoch mit Mikroklettsegmenten 360 ausgestattet, die mittels einer Trägerschicht 313 und einer Klebstoffschicht 314 an einer dehäsiven Beschichtung 316 in Gestalt einer Silikonschicht 317 einer Trägerbahn 303 des vierten Halbzeugstreifens 301 angeklebt sind. Somit weist der vierte Halbzeugstreifen 301 keinen durchgängigen Permanentbereich 321 an seiner Rückseite 320 auf, vielmehr ist der Permanentbereich 321 auch hier in einen ersten seitlichen Klebstoffauftragsstreifen 351, einen zweiten seitlichen Klebstoffauftragsstreifen 352 und einen mittleren Klebstoffauftragsstreifen 353 unterteilt. Der vierte Halbzeugstreifen 301 ist vorliegend ebenfalls symmetrisch hinsichtlich einer Symmetrieebene 305 ausgebildet und demnach in identische Windelverschlussbänder 309 entlang einer Trennlinie 306 trennbar.

Ist eines der Windelverschlussbänder 309 an einer Windel 323 gemäß der Darstellung nach der Figur 15 appliziert, das heißt mit seinem Permanentbereich 321 oberseitig 324 und unterseitig 325 der Windel 323 angeklebt und im Bereich einer Knicklinie 322 umgefaltet, ist hinter der Komponente 310 maximal eine Klebstofflage 327 angeordnet. In einem weiteren Teilbereich 361 hinter der Komponente 310 befindet sich überhaupt keine der Klebstofflagen 327 beziehungsweise 328 des Permanentbereiches 321. Durch diese spezielle Anordnung hinsichtlich des zweiten Klebstoffauftrags 319 an der Rückseite 320 kann insbesondere die zweite Klebstofflage 328 derart individuell gestaltet sein, dass der Bereich hinter der Komponente 310 des zweikomponentigen mechanischen Verschlusssystems weicher ausgebildet ist, wodurch die Mikroklettsegmente 360, welche hier ein Befestigungsgebiet 312 bereitstellen, flexibler mit einer hier nicht gezeigten Gegenkomponente des zweikomponentigen Verschlusssystems kommunizieren können. Somit befindet sich hinter dem Befestigungsgebiet 312 hinsichtlich der Trägerbahn 303 lediglich einer erste Trägerbahnlage 330 und eine zweite Trägerbahnlage 331.

Bei dem fünften Halbzeugstreifen 401 (siehe Figur 16) ist ein Bereich hinsichtlich Klebstoffaussparungen 450 (hier nur exemplarisch beziffert) in einem Permanentbereich 421 des fünften Halbzeugstreifens 401 mit einer Vielzahl 462 an Klebstoffauftragsstreifen ausgestattet, so dass im applizierten Zustand eines Windelverschlussbandes 409 (siehe Figur 17) ebenfalls ein nicht durchgängiger Permanentbereich 421 hinter einem Befestigungsgebiet 412 vorhanden ist, wodurch eine Komponente 410 eines zweikomponentigen mechanischen Verschlusssystems ebenfalls weicher ausgebildet werden kann.

Ansonsten ist der fünfte Halbzeugstreifen 401 ähnlich ausgebildet wie die vorstehend erläuterten Halbzeugstreifen, so dass auch das Windelverschlussband 409 ähnlich ausgebildet ist. Der fünfte Halbzeugstreifen 401 ist hinsichtlich einer Symmetrieebene 405 in einen ersten Halbzeugstreifennutzen 407 und in einen zweiten Halbzeugstreifennutzen 408 unterteilbar, wodurch der fünfte Halbzeugstreifen 401 entlang einer Trennlinie 406 in zwei identische Windelverschlussbänder 409 unterteilbar ist.

So umfasst der fünfte Halbzeugstreifen 401 ebenfalls eine Trägerbahn 403, welche hinsichtlich der Befestigungsseite 415 mit einer dehäsiven Beschichtung 416 ausgestattet ist, auf welcher jeweils die Komponente 410 des Windelverschlussbandes 409 mittels einer Trägerschicht 413 und einer Klebstoffschicht 414 aufgeklebt ist.

Das Windelschlussband 409 wird an einer Knicklinie 422 um ein Windelohr 426 gelegt, so dass es gleichzeitig an einer Oberseite 424 und an einer Unterseite 425 der Windel 423 befestigt werden kann. Hierbei ist eine erste Klebstofflage 427 des Permanentbereiches 421 an der Oberseite 424 und eine zweite Klebstofflage 428 des Permanentbereiches 421 an einer Unterseite 425 befestigt. Beidseits der Windel 323 befindet sich jeweils eine Trägerbahnlage 430 beziehungsweise 431. Somit ist die Trägerbahn 403 des Windelverschlussbandes 409 mehrlagig hinter dem Befestigungsgebiet 412 ausgebildet.

Wie insbesondere beispielsweise anhand der Figuren 2 bis 6, 8, 10 bis 13, 15 und 17 unmittelbar ersichtlich, kann die Trägerbahn 3, 103, 203, 303, 403 an der Knicklinie 22, 122, 222. 322, 422 oder auch näher an dem Befestigungsgebiet 12, 112, 212, 312, 412 durchtrennt werden, was je nach konkreter Verfahrensführung bei der Applikation vor, während oder nach der Applikation erfolgen kann. Hierdurch können insbesondere bezüglich der Ebene 32 symmetrische Trägerbahnanordnungen einfach bereitgestellt werden.

**Bezugszeichenliste:**

| | | | |
|---|---|---|---|
| 1 | erster Halbzeugstreifen | 29 | Teilgebiet |
| 2 | Trägerband | 30 | erste Trägerbahnlage |
| 3 | Trägerbahn | 31 | zweite Trägerbahnlage |
| 4 | Papierebene | 32 | Ebene |
| 5 | Symmetrieebene | 40 | erste Befestigungsvariante |
| 6 | Trennlinie | 41 | Ende |
| 7 | erster Halbzeugstreifennutzen | 42 | Scheitelbereich |
| 8 | zweiter Halbzeugstreifennutzen | 43 | zweite Befestigungsvariante |
| 9 | Windelverschlussband | 44 | dritte Befestigungsvariante |
| 10 | Komponente eines zweikomponentigen mechanischen Verschlusssystems | 45 | Benutzerbereich |
| | | 46 | Befestigungsbereich |
| | | 47 | Grenze |
| 11 | Haken | 48 | vierte Befestigungsvariante |
| 12 | Befestigungsgebiet | 49 | fünfte Befestigungsvariante |
| 13 | Trägerschicht | 101 | zweiter Halbzeugstreifen |
| 14 | Klebstoffschicht | 102 | Trägerband |
| 15 | Befestigungsseite | 103 | Trägerbahn |
| 16 | dehäsive Beschichtung | 104 | Papierebene |
| 17 | Silikonschicht | 105 | Symmetrieebene |
| 18 | erster Klebstoffauftrag | 106 | Trennlinie |
| 19 | zweiter Klebstoffauftrag | 107 | erster Halbzeugstreifennutzen |
| 20 | Rückseite | 108 | zweiter Halbzeugstreifennutzen |
| 21 | Permanentbereich | 109 | Windelverschlussband |
| 22 | Knicklinie | 110 | Komponente eines zweikomponentigen mechanischen Verschlusssystems |
| 23 | Windel | | |
| 24 | Oberseite | | |
| 25 | Unterseite | 111 | Haken |
| 26 | Windelohr | 112 | Befestigungsgebiet |
| 27 | erste Klebstofflage | 113 | Trägerschicht |
| 28 | zweite Klebstofflage | 114 | Klebstoffschicht |
| 115 | Befestigungsseite | 211 | Haken |
| 116 | dehäsive Beschichtung | 212 | Befestigungsgebiet |
| 118 | erster Klebstoffauftrag | 213 | Trägerschicht |
| 119 | zweiter Klebstoffauftrag | 214 | Klebstoffschicht |
| 120 | Rückseite | 215 | Befestigungsseite |
| 121 | Permanentbereich | 216 | dehäsive Beschichtung |
| 122 | Knicklinie | 217 | Silikonschicht |
| 123 | Windel | 219 | zweiter Klebstoffauftrag |
| 124 | Oberseite | 220 | Rückseite |
| 125 | Unterseite | 221 | Permanentbereich |
| 126 | Windelohr | 222 | Knicklinie |
| 127 | erste Klebstofflage | 223 | Windel |
| 128 | zweite Klebstofflage | 224 | Oberseite |
| 129 | Teilgebiet | 225 | Unterseite |
| 130 | erste Trägerbahnlage | 226 | Windelohr |
| 131 | zweite Trägerbahnlage | 227 | erste Klebstofflage |
| 150 | Klebstoffaussparungen | 228 | zweite Klebstofflage |
| 151 | erster seitlicher Klebstoffauftragsstreifen | 230 | erste Trägerbahnlage |
| | | 231 | zweite Trägerbahnlage |
| 152 | zweiter seitlicher Klebstoffauftragsstreifen | 245 | Benutzerbereich |
| | | 246 | Befestigungsbereich |
| 153 | mittlerer Klebstoffauftragsstreifen | 247 | Grenze |
| 154 | Befestigungsvariante | 260 | Mikroklettsegmente |
| 201 | dritter Halbzeugstreifen | 301 | vierter Halbzeugstreifen |
| 203 | Trägerbahn | 303 | Trägerbahn |
| 205 | Symmetrieebene | 305 | Symmetrieebene |
| 206 | Trennlinie | 306 | Trennlinie |
| 207 | erster Halbzeugstreifennutzen | 307 | erster Halbzeugstreifennutzen |
| 208 | zweiter Halbzeugstreifennutzen | 308 | zweiter Halbzeugstreifennutzen |
| 209 | Windelverschlussband | 309 | Windelverschlussband |
| 210 | Komponente | 310 | Komponente |
| 312 | Befestigungsgebiet | 410 | Komponente |
| 313 | Trägerschicht | 412 | Befestigungsgebiet |
| 314 | Klebstoffschicht | 413 | Trägerschicht |
| 316 | dehäsive Beschichtung | 414 | Klebstoffschicht |
| 317 | Silikonschicht | 415 | Befestigungsseite |
| 319 | zweiter Klebstoffauftrag | 416 | dehäsive Beschichtung |
| 320 | Rückseite | 421 | Permanentbereich |
| 321 | Permanentbereich | 422 | Knicklinie |
| 322 | Knicklinie | 423 | Windel |
| 323 | Windel | 424 | Oberseite |
| 324 | Oberseite | 425 | Unterseite |
| 325 | Unterseite | 426 | Windelohr |
| 327 | erste Klebstofflage | 427 | erste Klebstofflage |
| 328 | zweite Klebstofflage | 428 | zweite Klebstofflage |
| 330 | erste Trägerbahnlage | 430 | erste Trägerbahnlage |
| 331 | zweite Trägerbahnlage | 431 | zweite Trägerbahnlage |
| 350 | Klebstoffaussparungen | 450 | Klebstoffaussparungen |
| 351 | erster seitlicher Klebstoffauftragsstreifen | 462 | Vielzahl an Klebstoffauftragsstreifen |
| 352 | zweiter seitlicher Klebstoffauftragsstreifen | | |
| 353 | mittlerer Klebstoffauftragsstreifen | | |
| 360 | Mikroklettsegmente | | |
| 361 | weiterer Teilbereich | | |
| 401 | fünfter Halbzeugstreifen | | |
| 403 | Trägerbahn | | |
| 405 | Symmetrieebene | | |
| 406 | Trennlinie | | |
| 407 | erster Halbzeugstreifennutzen | | |
| 408 | zweiter Halbzeugstreifennutzen | | |
| 409 | Windelverschlussband | | |

## Patentansprüche

1. Windel (23) mit einem Windelverschlussband (9) mit einer Befestigungsseite (15) und mit einer Rückseite (20), wobei auf der Befestigungsseite (15) in wenigstens einem Befestigungsgebiet (12) eine Komponente (10) eines zweikomponentigen Verschlusssystems vorgesehen ist, die auf einer Trägerbahn (3) angeordnet ist, und wobei das Windelverschlussband (9) in einem Permanentbereich (21) an einer Windel (23) zu befestigen ist, **dadurch gekennzeichnet, dass** an der Rückseite (20) Klebstoff (19), mittels dessen das Windelverschlussband (9) dauerhaft befestigt werden kann, vorgesehen ist und dass die Trägerbahn (3) zumindest hinter einem Teilgebiet (29) des Befestigungsgebietes (12) und zumindest in dem Permanentbereich (21) mehrlagig ausgebildet ist, um so den Permanentbereich (21) hinter dem Befestigungsgebiet (12) vorzusehen und eine Lage (30) der Trägerbahn (3) vor und eine Lage (31) der Trägerbahn (3) hinter der Windel (23), insbesondere einem Windelohr (26) der Windel (23), anzuordnen.

2. Windel (23) nach Anspruch 1, **dadurch gekennzeichnet, dass** hinter dem Befestigungsgebiet (12) wenigstens teilweise Klebstoff (19) vorgesehen ist, der zwei Trägerbahnlagen (30, 31) direkt oder indirekt miteinander verbindet.

3. Windel (23) nach Anspruch 1 oder 2, **dadurch gekennzeichnet, dass** in dem Permanentbereich (21) wenigstens eine Lage (30) der Trägerbahn (3) vor und eine Lage (31) der Trägerbahn (3) hinter der Windel (23) angeordnet ist.

4. Windel (23) nach einem der vorstehenden Ansprüche, **dadurch gekennzeichnet, dass** die Trägerbahn (3) einstückig ausgebildet und an einer Knicklinie (22) umgefaltet ist.

5. Windel (23) nach einem der vorstehenden Ansprüche, **dadurch gekennzeichnet, dass** zwei Lagen (30, 31) der Trägerbahn (3) identisch aber bezüglich einer zwischen den Lagen (30, 31) liegenden Ebene (32) spiegelsymmetrisch ausgebildet sind.

6. Windelverschlussband (9) mit einer Befestigungsseite (15) und mit einer Rückseite (20), wobei auf der Befestigungsseite (15) in wenigstens einem Befestigungsgebiet (12) eine Komponente (10) eines zweikomponentigen Verschlusssystems vorgesehen ist, die auf einer Trägerbahn (3) angeordnet ist, und wobei die Trägerbahn (3) auf ihrer Befestigungsseite (15) eine dehäsive Beschichtung (16) aufweist, **dadurch gekennzeichnet, dass** Bestandteile der dehäsiven Beschichtung (16) hinter der Komponente (10) des zweikomponentigen Verschlusssystems zu finden sind.

7. Windelverschlussband (9) nach Anspruch 6, **dadurch gekennzeichnet, dass** die dehäsive Beschichtung (16) bis hinter die Komponente (10) des zweikomponentigen Verschlusssystems reicht.

8. Windel (23) nach einem der Ansprüche 1 bis 5 bzw. Windelverschlussband (9) nach Anspruch 6 oder 7, **dadurch gekennzeichnet, dass** die Trägerbahn (3) eine textile Trägerbahn ist und die Komponente (10) des zweikomponentigen Verschlusssystems über eine Klebstoffschicht (14) mit der textilen Trägerbahn (3) verbunden und die Klebstoffschicht (14) in Zwischenräume einer textilen Trägerbahn (3) eingedrungen ist.

9. Windel (23), insbesondere auch nach einem der Ansprüche 1 bis 5, mit einem Windelverschlussband (9) nach Anspruch 6 oder 7.

10. Halbzeugstreifen (1) umfassend eine Trägerbahn (3) mit einer Befestigungsseite (15) und mit einer Rückseite (20), bei welchem auf der Befestigungsseite (15) der Trägerbahn (3) eine Komponente (10) eines zweikomponentigen Verschlusssystems angeordnet ist, **dadurch gekennzeichnet, dass** auf der Rückseite (20) der Trägerbahn (3) eine einen Permanentbereich (21) ausbildende Klebstoffbeschichtung (19) durchgängig vorgesehen ist und dass der Halbzeugstreifen (1) eine Knicklinie (22) aufweist, wobei die Komponente (10) niemals auf der Knicklinie (22) liegt.

11. Halbzeugstreifen (1), insbesondere auch nach Anspruch 10, mit einer Befestigungsseite (15) und mit einer Rückseite (20), wobei auf der Befestigungsseite (15) in wenigstens einem Befestigungsgebiet (12) eine Komponente (10) eines zweikomponentigen Verschlusssystems vorgesehen ist, die auf einer Trägerbahn (3) angeordnet ist, und wobei die Trägerbahn (3) auf ihrer Befestigungsseite (15) eine dehäsive Beschichtung (16) aufweist, **dadurch gekennzeichnet, dass** Bestandteile der dehäsiven Beschichtung (16) hinter der Komponente (10) des zweikomponentigen Verschlusssystems zu finden sind.

12. Halbzeugstreifen (1) nach Anspruch 11, **dadurch gekennzeichnet, dass** die dehäsive Beschichtung (16) bis hinter die Komponente (10) des zweikomponentigen Verschlusssystems reicht.

13. Halbzeugstreifen (1) nach Anspruch 11 oder 12, **dadurch gekennzeichnet, dass** die Trägerbahn (3) eine textile Trägerbahn ist und die Komponente (10) des zweikomponentigen Verschlusssystems über eine Klebstoffschicht (14) mit der textilen Trägerbahn verbunden und die Klebstoffschicht (14) in Zwischenräume der textilen Trägerbahn eingedrungen ist.

14. Halbzeugstreifen (1) nach einem der Ansprüche 10 bis 13, **dadurch gekennzeichnet, dass** zwei voneinander beabstandete Bereiche jeweils eine Komponente (10) des zweikomponentigen Verschlusssystems tragen und mittig zwischen diesen Bereichen eine Symmetrieebene (5) liegt, bezüglich welcher der Halbzeugstreifen (1) symmetrisch ausgebildet ist.

15. Windelverschlussband (9) bzw. Halbzeugstreifen (1) nach einem der vorstehenden Ansprüche, **dadurch gekennzeichnet, dass** die Komponente (10) des zweikomponentigen Verschlusssystems eine Komponente (10) eines mechanischen Verschlusssystems ist.

16. Windelverschlussband (9) bzw. Halbzeugstreifen (1) nach Anspruch 15, **dadurch gekennzeichnet, dass** die Komponente (10) des mechanischen Verschlusssystems eine Hakenkomponente (11) eines Haken-Schlaufen-Verschlusssystems ist.

17. Verfahren zum Applizieren eines Windelverschlussbandes (9) an einer Windel (23), wobei das Windelverschlussband (9) von einem Halbzeugstreifen (1) senkrecht zu einer Maschinenrichtung abgetrennt und an der Windel (23) appliziert wird, **dadurch gekennzeichnet, dass** eine Trägerbahn (3) des Windelverschlussbandes (9), auf welcher eine Komponente (10) eines zweikomponentigen Verschlusssystems angeordnet ist, umgefaltet und beidseits an der Windel (23) permanent befestigt wird.

18. Verfahren nach Anspruch 17, **dadurch gekennzeichnet, dass** vor, während oder nach dem Umfalten die Trägerbahn (3) durchtrennt wird.

19. Verfahren zum Herstellen eines Halbzeugstreifens (1) mit einer Befestigungsseite (15) und mit einer Rückseite (20), wobei auf der Befestigungsseite (15) in wenigstens einem Befestigungsgebiet (12) eine Komponente (10) eines zweikomponentigen Verschlusssystems vorgesehen ist, die über eine Klebstoffschicht (14) mit einer Trägerbahn (3), welche auf der Befestigungsseite (15) mit einer dehäsiven Beschichtung (16) versehen ist, verbunden ist, **dadurch gekennzeichnet, dass** die Klebstoffschicht (14) wenigstens teilweise auf die dehäsive Beschichtung (16) aufgebracht und unter zusätzlichem Energieeintrag mit der Trägerbahn (3) verbunden wird.

20. Verfahren nach Anspruch 19, **dadurch gekennzeichnet, dass** die Klebstoffschicht (14) in verformbarem, flüssigem und/oder heißem Zustand mit der Trägerbahn (3) verbunden wird.

21. Verfahren nach Anspruch 19 oder 20, **dadurch gekennzeichnet, dass** die Klebstoffschicht (14) mit der Trägerbahn (3) verpresst wird.

## Claims

1. A diaper (23) with a diaper fastener (9) with a fastening side (15) and with a rear side (20), wherein on the fastening side (15) in at least one fastening region (12) a component (10) of a two-component fastening system is provided, which is arranged on a carrier web (3), and wherein the diaper fastener (9) is to be fastened in a permanent region (21) on a diaper (23), **characterized in that** on the rear side (20) adhesive (19) is provided, by means of which the diaper fastener (9) can be permanently fastened, and that the carrier web (3) at least behind a partial region (29) of the fastening region (12) and at least in the permanent region (21) is formed so as to be multi-layered, in order to thus provide the permanent region (21) behind the fastening region (12) and to arrange a layer (30) of the carrier web (3) in front and a layer (31) of the carrier web (3) behind the diaper (23), in particular a diaper ear (26) of the diaper (23).

2. The diaper (23) according to Claim 1, **characterized in that** behind the fastening region (12), adhesive (19) is provided at least partially, which adhesive connects two carrier web layers (30, 31) to one another directly or indirectly.

3. The diaper (23) according to Claim 1 or 2, **characterized in that** in the permanent region (21) at least one layer (30) of the carrier web (3) is arranged in front of, and one layer (31) of the carrier web (3) is arranged behind the diaper (23).

4. The diaper (23) according to one of the preceding claims, **characterized in that** the carrier web (3) is formed in one piece and is folded over on a bending line (22).

5. The diaper (23) according to one of the preceding claims, **characterized in that** two layers (30, 31) of the carrier web (3) are formed identically but mirror-symmetrically with respect to a plane (32) lying between the layers (30, 31).

6. A diaper fastener (9), with a fastening side (15) and with a rear side (20), wherein on the fastening side (15) in at least one fastening region (12) a component (10) of a two-component fastening system is provided, which is arranged on a carrier web (3), and wherein the carrier web (3) has a dehesive coating (16) on its fastening side (15), **characterized in that** component parts of the dehesive coating (16) are to be found behind the component (10) of the two-component fastening system.

7. The diaper fastener (9) according to Claim 6, **characterized in that** the dehesive coating (16) extends to behind the component (10) of the two-component fastening system.

8. The diaper (23) according to one of Claims 1 to 5 or respectively diaper fastener (9) according to Claim 6 or 7, **characterized in that** the carrier web (3) is a textile carrier web and the component (10) of the two-component fastening system is connected with the textile carrier web (3) via an adhesive layer (14), and the adhesive layer (14) has penetrated into intermediate spaces of a textile carrier web (3).

9. The diaper (23), in particular also according to one of Claims 1 to 5, with a diaper fastener (9) according to Claim 6 or 7.

10. A semi-finished strip (1) comprising a carrier web (3) with a fastening side (15) and with a rear side (20), in which on the fastening side (15) of the carrier web (3) a component (10) of a two-component fastening system is arranged, **characterized in that** on the rear side (20) of the carrier web (3) an adhesive coating (19), forming a permanent region (21), is provided continuously and that the semi-finished strip (1) has a bending line (22), wherein the component (10) never lies on the bending line (22).

11. A semi-finished strip (1), in particular also according to Claim 10, with a fastening side (15) and with a rear side (20), wherein on the fastening side (15) a component (10) of a two-component fastening system is provided in at least one fastening region (12), which component is arranged on a carrier web (3), and wherein the carrier web (3) has a dehesive coating (16) on its fastening side (15), **characterized in that** component parts of the dehesive coating (16) are to be found behind the component (10) of the two-component fastening system.

12. The semi-finished strip (1) according to Claim 11, **characterized in that** the dehesive coating (16) extends to behind the component (10) of the two-component fastening system.

13. The semi-finished strip (1) according to Claim 11 or 12, **characterized in that** the carrier web (3) is a textile carrier web and the component (10) of the two-component fastening system is connected with the textile carrier web via an adhesive layer (14), and the adhesive layer (14) has penetrated into intermediate spaces of the textile carrier web.

14. The semi-finished strip (1) according to one of Claims 10 to 13, **characterized in that** two regions, spaced apart from one another, carry respectively a component (10) of the two-component fastening system and a symmetry plane (5) lies centrally between these regions, with respect to which symmetry plane the semi-finished strip (1) is formed symmetrically.

15. The diaper fastener (9) or respectively semi-finished strip (1) according to one of the preceding claims, **characterized in that** the component (10) of the two-component fastening system is a component (10) of a mechanical fastening system.

16. The diaper fastener (9) or respectively semi-finished strip (1) according to Claim 15, **characterized in that** the component (10) of the mechanical fastening system is a hook component (11) of a hook-and-loop fastening system.

17. A method for applying a diaper fastener (9) on a diaper (23), wherein the diaper fastener (9) is separated from a semi-finished strip (1) perpendicularly to a machine direction and is applied on the diaper (23), **characterized in that** a carrier web (3) of the diaper fastener (9), on which a component (10) of a two-component fastening system is arranged, is folded over and is fastened permanently on both sides on the diaper (23).

18. The method according to Claim 17, **characterized in that** before, during or after the folding over, the carrier web (3) is separated through.

19. A method for producing a semi-finished strip (1) with a fastening side (15) and with a rear side (20), wherein on the fastening side (15) in at least one fastening region (12) a component (10) of a two-component fastening system is provided, which is connected via an adhesive layer (14) with a carrier web (3), which is provided on the fastening side (15) with a dehesive coating (16), **characterized in that** the adhesive layer (14) is applied at least partially onto the dehesive coating (16) and is connected, with additional application of energy, with the carrier web (3) .

20. The method according to Claim 19, **characterized in that** the adhesive layer (14) is connected in deformable, liquid and/or hot state with the carrier web (3).

21. The method according to Claim 19 or 20, **characterized in that** the adhesive layer (14) is pressed with the carrier web (3).

## Revendications

1. Couche-culotte (23) avec une bande de fermeture de couche-culotte (9) avec un côté de fixation (15) et un côté arrière (20), dans laquelle, sur le côté de fixation (15), dans au moins une zone de fixation (12), on prévoit un composant (10) d'un système de fermeture à deux composants, lequel composant est disposé sur une bande support (3), et dans laquelle la bande de fermeture de couche-culotte (9) doit être fixée dans une zone permanente (21) à une couche-culotte (23), **caractérisée en ce que,** sur le côté arrière (20), un adhésif (19) est prévu au moyen duquel la bande de fermeture de couche-culotte (9) peut être fixée de manière durable et que la bande support (3) est réalisée en plusieurs couches au moins derrière une zone partielle (29) de la zone de fixation (12) et au moins dans la zone permanente (21) afin de prévoir ainsi la zone permanente (21) derrière la zone de fixation (12) et disposer une couche (30) de la bande support (3) devant, et une couche (31) de la bande support (3) derrière la couche-culotte (23), en particulier une oreille de couche-culotte (26) de la couche-culotte (23).

2. Couche-culotte (23) selon la revendication 1, **caractérisée en ce que,** derrière la zone de fixation (12), on prévoit au moins partiellement un adhésif (19) qui relie directement ou indirectement deux couches de bande support (30, 31) entre elles.

3. Couche-culotte (23) selon la revendication 1 ou 2, **caractérisée en ce que,** dans la zone permanente (21), au moins une couche (30) de la bande support (3) est disposée devant, et une couche (31) de la bande support (3) est disposée derrière la couche-culotte (23) .

4. Couche-culotte (23) selon l'une des revendications précédentes, **caractérisée en ce que** la bande support (3) est réalisée d'une seule pièce et est repliée au niveau d'une ligne de pliage (22).

5. Couche-culotte (23) selon l'une des revendications précédentes, **caractérisée en ce que** deux couches (30, 31) de la bande support (3) sont réalisées de manière identique mais avec une symétrie spéculaire compte tenu d'un plan (32) situé entre les couches (30, 31).

6. Bande de fermeture de couche-culotte (9) avec un côté de fixation (15) et un côté arrière (20), dans laquelle, sur le côté de fixation (15), dans au moins une zone de fixation (12), on prévoit un composant (10) d'un système de fermeture à deux composants, lequel composant est disposé sur une bande support (3), et dans laquelle la bande support (3) présente un revêtement non adhésif (16) sur son côté de fixation (15), **caractérisée en ce que** des éléments constitutifs du revêtement non adhésif (16) se trouvent derrière le composant (10) du système de fermeture à deux composants.

7. Bande de fermeture de couche-culotte (9) selon la revendication 6, **caractérisée en ce que** le revêtement non adhésif (16) va jusque derrière le composant (10) du système de fermeture à deux composants.

8. Couche-culotte (23) selon l'une des revendications 1 à 5 ou bande de fermeture de couche-culotte (9) selon la revendication 6 ou 7, **caractérisée en ce que** la bande support (3) est une bande support textile et le composant (10) du système de fermeture à deux composants est relié via une couche adhésive (14) à la bande support textile (3) et que la couche adhésive (14) a pénétré dans des espaces intermédiaires d'une bande support textile (3).

9. Couche-culotte (23), en particulier également selon l'une des revendications 1 à 5, avec une bande de fermeture de couche-culotte (9) selon la revendication 6 ou 7.

10. Ruban de produit semi-fini (1) comprenant une bande support (3) avec un côté de fixation (15) et avec un côté arrière (20), dans lequel, sur le côté de fixation (15) de la bande support (3), un composant (10) d'un système de fermeture à deux composants est disposé, **caractérisée en ce que,** sur le côté arrière (20) de la bande support (3), un revêtement adhésif (19) formant une zone permanente (21) est prévu d'un seul tenant et que le ruban de produit semi-fini (1) présente une ligne de pliage (22), dans lequel le composant (10) ne se situe jamais sur la ligne de pliage (22).

11. Ruban de produit semi-fini (1), en particulier également selon la revendication 10, avec un côté de fixation (15) et avec un côté arrière (20), dans lequel, sur le côté de fixation (15), dans au moins une zone de fixation (12), un composant (10) d'un système de fermeture à deux composants est prévu, lequel composant est disposé sur une bande support (3), et dans lequel la bande support (3) présente un revêtement non adhésif (16) sur son côté de fixation (15), **caractérisé en ce que** des éléments constitutifs du revêtement non adhésif (16) se trouvent derrière le composant (10) du système de fermeture à deux composants.

12. Ruban de produit semi-fini (1) selon la revendication 11, **caractérisée en ce que** le revêtement non adhésif (16) va jusque derrière le composant (10) du système de fermeture à deux composants.

13. Ruban de produit semi-fini (1) selon la revendication 11 ou 12, **caractérisée en ce que** la bande support (3) est une bande support textile et que le composant (10) du système de fermeture à deux composants est relié via une couche adhésive (14) à la bande support textile et que la couche adhésive (14) a pénétré dans des espaces intermédiaires de la bande support textile.

14. Ruban de produit semi-fini (1) selon l'une des revendications 10 à 13, **caractérisée en ce que** deux zones espacées l'une de l'autre portent respectivement un composant (10) du système de fermeture à deux composants et qu'un plan de symétrie (5) se trouve au milieu entre ces zones, les rubans de produit semi-fini (1) étant réalisés de manière symétrique par rapport audit plan.

15. Bande de fermeture de couche-culotte (9) ou ruban de produit semi-fini (1) selon l'une des revendications précédentes, **caractérisé(e) en ce que** le composant (10) du système de fermeture à deux composants est un composant (10) d'un système de fermeture mécanique.

16. Bande de fermeture de couche-culotte (9) ou ruban de produit semi-fini (1) selon la revendication 15, **caractérisé en ce que** le composant (10) du système de fermeture mécanique est un composant de crochet (11) d'un système de fermeture à crochet et boucle.

17. Procédé pour l'application d'une bande de fermeture de couche-culotte (9) sur une couche-culotte (23), dans lequel la bande de fermeture de couche-culotte (9) est détachée d'un ruban de produit semi-fini (1) perpendiculairement à une direction de machine et est appliquée sur la couche-culotte (23), **caractérisé en ce qu'**une bande support (3) de la bande de fermeture de couche-culotte (9), sur laquelle un composant (10) d'un système de fermeture à deux composants est disposé, est repliée et est fixée de façon permanente des deux côtés sur la couche-culotte (23).

18. Procédé selon la revendication 17, **caractérisé en ce que** la bande support (3) est sectionnée avant, pendant, ou après le pliage.

19. Procédé pour la fabrication d'un ruban de produit semi-fini (1) avec un côté de fixation (15) et avec un côté arrière (20), dans lequel, sur le côté de fixation (15), dans au moins une zone de fixation (12), on prévoit un composant (10) d'un système de fermeture à deux composants, lequel composant est relié via une couche adhésive (14) à une bande support (3) qui est dotée d'un revêtement non adhésif (16) sur le côté de fixation (15), **caractérisé en ce que** la couche adhésive (14) est appliquée au moins partiellement sur le revêtement non adhésif (16) et est reliée à la bande support (3) par apport d'énergie supplémentaire.

20. Procédé selon la revendication 19, **caractérisé en ce que** la couche adhésive (14) est reliée à la bande support (3) à l'état déformable, liquide et/ou chaud.

21. Procédé selon la revendication 19 ou 20, **caractérisé en ce que** la couche adhésive (14) est pressée avec la bande support (3).
